# EUROPEAN PATENT APPLICATION

(11) **EP 2 415 453 A1**
(43) Date of publication of application: **08.02.2012**
(21) Application number: 10758366.8
(22) Date of filing: 04.03.2010
(51) Int. Cl.: A61K 8/49, A23L 1/30, A61K 31/401, A61P 17/00, A61Q 19/02

(54) **SKIN-WHITENING AGENT AND COSMETIC METHOD FOR WHITENING SKIN**

(30) Priority: 31.03.2009 JP 2009088222
(71) Applicant: Shiseido Company, Ltd., Chuo-ku Tokyo 104-8010 (JP)
(72) Inventor: SUZUKI, Rikako, Yokohama-shi Kanagawa 224-8558 (JP); TOJO, Yosuke, Yokohama-shi Kanagawa 236-0004 (JP); MIZUMOTO, Chieko, Yokohama-shi Kanagawa 236-0004 (JP); HASEGAWA, Kiyotaka, Yokohama-shi Kanagawa 2248558 (JP); ASHIDA, Yutaka, Yokohama-shi Kanagawa 236-0004 (JP); HOSOI, Jun-ichi, Yokohama-shi Kanagawa 236-0004 (JP); SATO, Kiyoshi, Yokohama-shi Kanagawa 224-8558 (JP)
(74) Representative: Henkel, Breuer & Partner
(86) International application number: PCT/JP2010/053569
(87) International publication number: WO 2010/113590

(57) **Abstract**

The development of a skin-whitening agent which has a lower molecular weight, can be synthesized through a fewer steps and can be produced at low cost has been demanded. Thus, it is provided by the present invention a skin-whitening agent comprising one or more compounds selected from the group consisting of 4-cis-hydroxyproline and a derivative and/or a salt thereof, a skin-whitening composition for external formulation to the skin, a cosmetic composition and a pharmaceutical composition, each of which contains the skin-whitening agent of the invention, and a cosmetic method for whitening the skin, which comprises a step of administering one or more compounds selected from the group consisting of 4-cis-hydroxyproline and a derivative and/or a salt thereof. In the skin-whitening agent and the cosmetic method for whitening the skin of the invention, the 4-cis-hydroxyproline may be 4-cis-L-hydroxyproline and/or 4-cis-D-hydroxyproline.

## Description

### TECHNICAL FIELD

The present invention relates to a skin-whitening agent and a cosmetic method for whitening skin. Specifically, it relates to a skin-whitening agent comprising one or more compounds selected from the group consisting of 4-cis-hydroxyproline and a derivative and/or a salt thereof, and a cosmetic method for whitening the skin comprising a step of administering the skin-whitening agent.

### BACKGROUND ART

Melanin is a skin pigment that is produced by a melanocyte present near the basement membrane of an epidermis. Being surrounded by epidermal cells, it migrates with epidermal cells to skin surface, and eventually discarded as dirt from the skin. Pigment abnormality such as darkening of skin, lentigo, and speckles are caused by abnormal increase or deposition of melanin in the epidermis, and involvement of UV light, female hormones, and genetic causes has been suggested. In this connection, a skin-whitening agent for reducing pigment deposition in skin by inhibiting the production of melanin was developed (Patent Literatures 1 and 2, and Non-Patent Literatures 1 and 2).

For example, hydroquinone as a reducing agent has a skin-whitening effect and it is designated as quasi medicines for preventing pigmentation caused by UV light. Further, a compound having an inhibitory activity for tyrosinase, which is an enzyme to catalyze the initial two steps of the reaction for melanin synthesis, is considered as a useful skin-whitening agent. Until now, compounds such as methyl gentisic acid, benzimidazole, and resorcinol have been developed as a skin-whitening agent (Patent Literatures 1 and 2).

### PRIOR ART DOCUMENTS

Patent Document 1: US Patent No. 6,852,747
Patent Document 2: US Patent Application Publication No. 2009/0023792

### Non-Patent Documents

Non-Patent Document 1: Mutagenesis, 5: 263-266 (1990).
Non-Patent Document 2: Biochem. Pharmacol., 57: 663-672 (1999)

### DISCLOSURE OF THE INVENTION

### PROBLEMS TO BE SOLVED BY THE INVENTION

Hydroquinone is known to have an adverse effect of causing skin irritation, etc. and a mutagenic activity in Chinese hamster V79 cells (Non-Patent Document 1). Although methyl gentisic acid has no such mutagenic property, it has a molecular weight of 168, and therefore there has been a demand to develop a skin-whitening agent which has a lower molecular weight, can be synthesized through a fewer steps and can be produced at low cost. There is also a demand to develop a pharmaceutical product, a cosmetic product, and a food product each of which comprises the skin-whitening agent, and a cosmetic method for whitening the skin including a step of administering the skin-whitening agent.

### MEANS FOR SOLVING THE PROBLEMS

The present invention provides a skin-whitening agent containing one or more compounds selected from the group consisting of 4-cis-hydroxyproline and a derivative and/or a salt thereof.

According to the skin-whitening agent of the present invention, the 4-cis-hydroxyproline may be 4-cis-L-hydroxyproline and/or 4-cis-D-hydroxyproline.

The present invention provides a cosmetic composition containing the skin-whitening agent of the present invention.

The cosmetic composition of the present invention may be a formulation for external application.

The present invention provides a food composition containing the skin-whitening agent of the present invention.

The present invention provides a pharmaceutical composition containing the skin-whitening agent of the present invention.

The pharmaceutical composition of the present invention may be a formulation for external application.

The invention provides a cosmetic method for whitening the skin including a step of administering one or more compounds selected from the group consisting of 4-cis-hydroxyproline and a derivative and/or a salt thereof.

According to the cosmetic method for whitening the skin, the 4-cis-hydroxyproline may be 4-cis-L-hydroxyproline and/or 4-cis-D-hydroxyproline.

According to the cosmetic method for whitening the skin, the step of administering the compound may be external application on skin.

According to the cosmetic method for whitening the skin, the step of administering the compound may be oral administration.

The invention provides a use of one or more compounds selected from the group consisting of 4-cis-hydroxyproline and a derivative and/or a salt thereof for producing a pharmaceutical composition for treating a disorder accompanied by an abnormality in skin pigmentation. For the application for producing the pharmaceutical composition of the invention, 4-cis-hydroxyproline can be 4-cis-L-hydroxyproline and/or 4-cis-D-hydroxyproline.

Hydroxyproline has eight kinds of stereoisomers based on position of hydroxy group, i.e. position 3 or position 4 (position isomer), form of the stereoisomer like cis form or trans form (geometrical isomer), and form of proline like D form or L form (optical isomer). Hydroxyproline is generally present in nature as 4-trans-L-hydroxyproline and very limited presence of other stereoisomers is known. Herein below, structural formula of the geometrical isomers and optical isomers of 4-hydroxyproline is given.

[Formula 1]

In the detailed description, "hydroxyproline" includes all proline compounds in which position 3 or position 4 of the heterocycle in the L form or D form proline is hydroxylated. Among them, the compounds in which position 4 is hydroxylated include 4-cis-L-hydroxyproline, 4-cis-D-hydroxyproline, 4-trans-L-hydroxyproline and 4-trans-D-hydroxyproline. According to the invention, "4-cis-hydroxyproline" indicates any one of 4-cis-L-hydroxyproline only, 4-cis-D-hydroxyproline only, and 4-cis-L-hydroxyproline and 4-cis-D-hydroxyproline.

According to the present invention, "skin-whitening" or "whitens the skin" means that pigment abnormality in skin such as darkening, flecks, and speckles is removed, reduced, and/or prevented. The mechanism of skin whitening includes inhibition of melanocyte differentiation, inhibition of melanin production in melanocyte by inhibiting a melanin producing enzyme such as tyrosinase, degradation, reduction, and depigmentation, etc. of produced melanin, and promoted excretion of melanin in epidermis, but it is not limited thereto.

In the detailed description, a "disorder accompanying an abnormality in skin pigmentation" includes, but is not limited, ephelides, chloasma, pigmented nevus+, blue nevus, nevus of Ota, acquired dermal melanocytosis, acropigmentatio reticularis, acromelanosis progressiva, senile spots, chromatosis contact dermatitis, sunburn, pigmentation petaloides actinica, photoluekomelanoderma, chemical-pharmaceutical pigmentation, Addison's disease, pigmentation caused by pregnancy, and Crow-Fukase syndrome, etc.

According to the present invention, the derivative of 4-cis-hydroxyproline is defined as that an atom other than the hydrogen atom which constitutes 4-cis-hydroxyproline is covalently bonded to any one of atomic groups under the condition that the skin-whitening effect of 4-cis-hydroxyproline is not reduced. The any one of atomic groups includes, but is not limited to, a protective group such as N-phenyl acetyl group and 4,4'-dimethoxytrityl (DMT) group, biological macromolecules such as protein, peptide, sugar, lipid, and nucleic acid, a synthetic polymer such as polystyrene, polyethylene, polyvinyl, and polyester, and a functional group such as ester group. The ester group may include an aliphatic ester such as methyl ester and ethyl ester or an aromatic ester, for example.

According to the present invention, the salt of 4-cis-hydroxyproline means any salt such as metal salt and amine salt, etc. under the condition that the skin-whitening effect of 4-cis-hydroxyproline is not reduced. The metal salt may include an alkaline metal salt and an alkaline earth metal salt. The amine salt may include a triethylamine salt and a benzylamine salt.

As shown in the Examples below, 4-cis-hydroxyproline of the present invention as a simple body has, within the concentration range of 33.3 to 333 ppm or 11.1 to 33.3 ppm, an effect of reducing melanin content in cultured mouse B16 melanoma cells. As such, the amount of 4-cis-hydroxyproline contained in the pharmaceutical composition, cosmetic composition, and food composition of the invention may be any content as long as 4-cis-hydroxyproline itself within the concentration range above is delivered to a melanocyte in a skin tissue of a living organism. Where the composition of the invention is prepared as an external application, the content of 4-cis-hydroxyproline may be from 0.000015% by weight to 50% by weight compared to the total weight of the composition of the invention, or it may be within the range having an upper value of weight concentration at which the compound may be contained in maximum amount. Specifically, when the composition of the invention is an external formulation, the content of 4-cis-hydroxyproline is preferably 0.00003% by weight to 30% by weight, and most preferably 0.0003% by weight to 3% by weight. When the composition of the invention is a formulation for internal application, the content of 4-cis-hydroxyproline may be within the range of 0.00001% by weight to 100% by weight. When the composition of the invention is a formulation for internal application, the content of 4-cis-hydroxyproline is preferably 0.00002% by weight to 80% by weight, and most preferably, 0.0002% by weight to 60% by weight. The lower intake limit per day of 4-cis-hydroxyproline in the composition of the invention may be 0.01 ng, 0.1 ng, or 1 ng per kg of body weight.

The cosmetic composition of the invention may be formulated appropriately as required with other components that are used for a cosmetic product such as quasi drugs or an external formulation for skin such as pharmaceuticals under the condition that the skin-whitening effect of 4-cis-hydroxyproline is not reduced. Examples of the other components (i.e. optionally added components) include an oil, a detergent, powder, a colorant, water, alcohols, a viscosity improver, a chelating agent, silicone, an anti-oxidant, a UV absorbing agent, a moisturizing agent, a flavouring agent, variety of pharmaceutical compounds, a preservative, a pH adjustment agent, and a neutralizing agent or the such as.

The cosmetic composition of the invention may be any one of conventionally used external formulations for skin and those used as a cosmetic composition such as ointment, cream, emulsion, lotion, pack, and bathing product, and the formulation type is not specifically limited.

The food composition of the invention may include, in addition to 4-cis-hydroxyproline itself, a salt of 4-cis-hydroxyproline, and/or its derivative which can release 4-cis-hydroxyproline by an enzyme for metabolizing a drug in a living body, a component which is allowed for a food product such as a flavoring agent, a coloring agent, and a preservative under the condition that the skin-whitening effect of 4-cis-hydroxyproline is not inhibited.

Examples of the food composition of the invention include, but is not limited to, any of those conventionally used as a food composition such as beverages, gummy candy, candy, and biscuit.

The pharmaceutical composition of the invention may contain, in addition to 4-cis-hydroxyproline itself, a salt of 4-cis-hydroxyproline, and/or its derivative which can release 4-cis-hydroxyproline by an enzyme for metabolizing a drug in a living body, one or more of other components which are effective for treating a disorder and/or pharmaceutically acceptable additives under the condition that the skin-whitening effect of 4-cis-hydroxyproline is not inhibited. The additives include a diluting agent, a swelling agent, a binding agent, adhesives, a lubricating agent, a fluidity promoting agent, a plasticizing agent, a disintegrating agent, a carrier solvent, a buffer agent, a coloring material, a flavor, a sweetening agent, a preservative, a stabilizing agent, an adsorbing agent, and other additives for pharmaceuticals that are known to those skilled in the art, but they are not limited thereto.

### BRIEF DESCRIPTION OF THE DRAWINGS

FIG. 1A is a graph illustrating the effect of 4-trans-L-hydroxyproline on cell growth and melanin content;
FIG. 1B is a graph illustrating the effect of 4-trans-D-hydroxyproline on cell growth and melanin content;
FIG. 1C is a graph illustrating the effect of 4-cis L-hydroxyproline on cell growth and melanin content;
FIG. 1D is a graph illustrating the effect of 4-cis-D-hydroxyproline on cell growth and melanin content;
FIG. 2A is a graph illustrating the effect of 4-trans-L-hydroxyproline on cell growth and melanin content reduction;
FIG. 2B is a graph illustrating the effect of 4-cis-D-hydroxyproline on cell growth and melanin content reduction;
FIG. 2C is a graph illustrating the effect of a mixture solution of 4-trans-L-hydroxyproline and 4-cis-D-hydroxyproline on cell growth and melanin content reduction;
FIG. 3A is a graph illustrating the effect of 4-trans-L-hydroxyproline on cell growth and melanin content reduction;
FIG. 3B is a graph illustrating the effect of 4-cis-D-hydroxyproline on cell growth and melanin content reduction; and
FIG. 3C is a graph illustrating the effect of a mixture solution of 4-trans-L-hydroxyproline and 4-cis-D-hydroxyproline on cell growth and melanin content reduction, wherein the mixture solution is prepared at various ratio.

### DESCRIPTION OF EMBODIMENTS

The Examples given below are only to illustrate the invention, and the scope of the invention is not limited by them. The scope of the invention is defined only by the working of the claims.

### Example 1

### Cell culture

Mouse B16 melanoma cell was used. The cells were inoculated at 1×10⁵ cells/well to a 6-well plate, and cultured with using a commercially available medium (trade name: EAGLE MEM, manufactured by Nissui Pharmaceutical Co., Ltd.) supplemented with 10% fetal bovine serum. The cells were cultured at 37°C, 5% CO₂ and under completely saturated water-vapor atmosphere.

### Reagents

Among the hydroxyprolines used for the Examples of the invention, 4-cis-L-hydroxyproline, 4-cis-D-hydroxyproline and 4-trans-L-hydroxyproline were purchased from Sigma-Aldrich Japan, and 4-trans-D-hydroxyproline was purchased from Bachem.

### Addition of hydroxyproline

For determining the effect of hydroxyproline on cell growth and melanin content reduction, after culturing the cells for 24 hours, stock solutions of 4-trans-L-hydroxyproline, 4-trans-D-hydroxyproline, 4-cis-L-hydroxyproline or 4-cis-D-hydroxyproline were prepared and added in the same volume to the cells, further cultured for 3 days and used for the experiment. Meanwhile, as a control group, the same volume of water was added.

### Quantification of the number of cells

3 days after the addition of a solution of test compound, the medium was removed by suction. Eagle's-MEM containing the solution of 10% alamarBlue (trade name: BIOSOURCE, manufactured by Biosource International) was added and the reaction was allowed to occur at 37°C. Thirty minutes later, the fluorescence was measured with excitation wavelength of 544 nm and measurement wavelength of 590 nm. By having the obtained number as a relative value of the cell number, the ratio of number of cells (i.e. % number of cells) for a group added with the test compound compared to a group not added with the test compound (i.e. only the solvent was added) was calculated. Higher % number of cells indicates lower cell toxicity. When it is less than 80%, it was determined as "toxic."

### Results of quantifying the number of cells

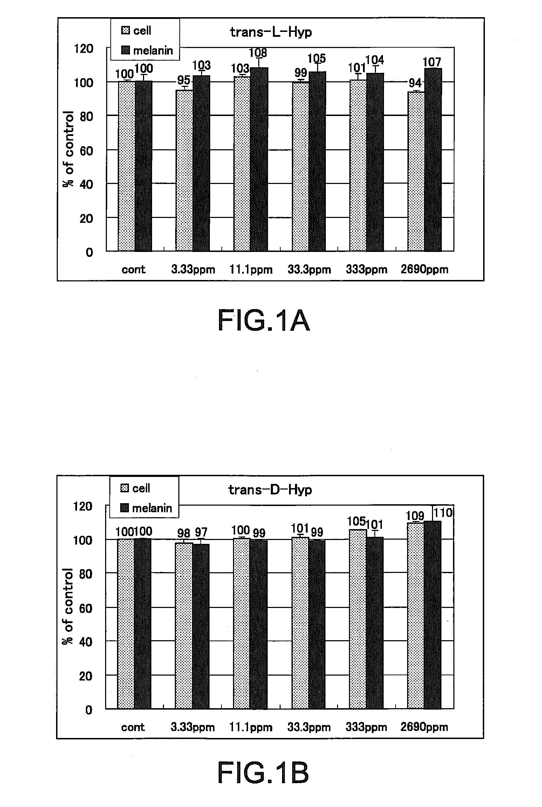

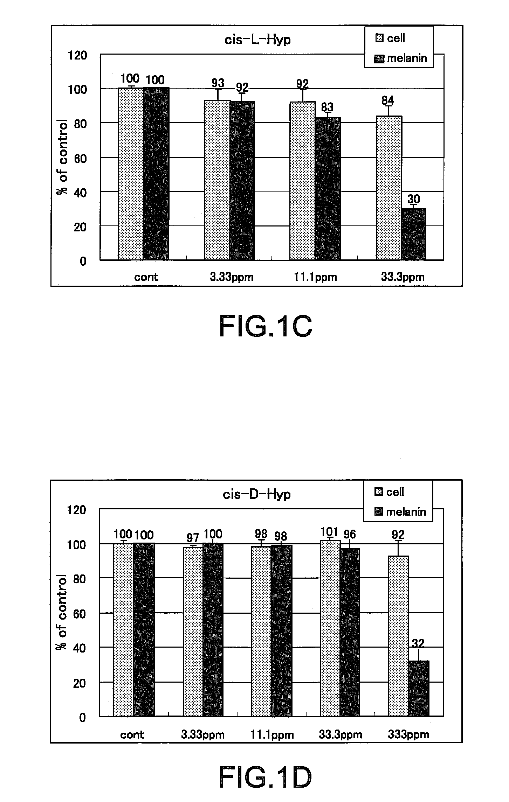

In FIGS. 1A to 1D (hatched bars), the results for examining the number of mouse B16 melanoma cells that are added with 4-trans-L-hydroxyproline, 4-trans-D-hydroxyproline, 4-cis-L-hydroxyproline, or 4-cis-D-hydroxyproline at various concentrations were given when the control was represented as 100%. FIG. 1A indicates the number of cells after adding 4-trans-L-hydroxyproline at various concentrations when the control was represented as 100%, and it was 95% at 3.33 ppm, 103% at 11.1 ppm, 99% at 33.3 ppm, 101% at 333 ppm, and 94% at 2690 ppm. FIG. 1B indicates the number of cells after adding 4-trans-D-hydroxyproline at various concentrations when the control was represented as 100%, and it was 98% at 3.33 ppm, 100% at 11.1 ppm, 101% at 33.3 ppm, 105% at 333 ppm, and 109% at 2690 ppm. FIG. 1C indicates the number of cells after adding 4-cis-L-hydroxyproline at various concentrations when the control was represented as 100%, and it was 93% at 3.33 ppm, 92% at 11.1 ppm, and 84% at 33.3 ppm. FIG. 1D indicates the number of cells after adding 4-cis-D-hydroxyproline at various concentrations when the control was represented as 100%, and it was 97% at 3.33 ppm, 98% at 11.1 ppm, 101% at 33.3 ppm, and 92% at 333 ppm. From the results above, the numbers of cells cultured under experimental conditions were represented as percentage of the number of cells cultured in the control condition (100%). The number of cells cultured up to 2690 ppm for 4-trans-L-hydroxyproline and 4-trans-D-hydroxyproline, up to 33.3 ppm for 4-cis-L-hydroxyproline and 333 ppm for 4-cis-D-hydroxyproline, were not significantly different from the number of cells cultured under the control conditions. Thus the above mentioned concentrations of hydroxyproline isomers are not demonstrated to affect survival of mouse B16 melanoma cells.

### Quantification of melanin

The medium was removed by suction. After washing three times with the buffer (phosphate buffer solution 50 mM, pH 6.8), the cells were lysed by addition of 1 N NaOH, and the absorbance was measured at 475 nm. By having the obtained value as a relative value of the melanin content, the ratio of melanin content (%) for a group added with the test compound compared to a group not added with the test compound (i.e. only the solvent was added) was calculated. Lower ratio of melanin content means the higher inhibition effect on melanin production.

### Determination of melanin content

By using the measured value of melanin content that was obtained according to the measurement method above, percentage ratio of the melanin content was calculated by dividing the melanin content in the cells added with 4-trans-L-hydroxyproline, 4-trans-D-hydroxyproline, 4-cis-L-hydroxyproline, or 4-cis-D-hydroxyproline by the melanin content in the cells for control experiment, and the resulting value was taken as a ratio of melanin content.

### Results of melanin quantification

In FIGS. 1A to 1D (solid bars), the experiment results for examining the reduction in melanin content in mouse B16 melanoma cells that were added with 4-trans-L-hydroxyproline, 4-trans-D-hydroxyproline, 4-cis-L-hydroxyproline, or 4-cis-D-hydroxyproline at various concentrations are shown. FIG. 1A indicates the melanin content ratio after adding 4-trans-L-hydroxyproline at various concentrations, wherein the ratio was 103% at 3.33 ppm, 108% at 11.1 ppm, 105% at 33.3 ppm, 104% at 333 ppm, and 107% at 2690 ppm. FIG. 1B indicates the melanin content ratio after adding 4-trans-D-hydroxyproline at various concentrations, wherein the ratio was 97% at 3.33 ppm, 99% at 11.1 ppm, 99% at 33.3 ppm, 101% at 333 ppm, and 110% at 2690 ppm. FIG. 1C indicates the melanin content ratio after adding 4-cis-L-hydroxyproline at various concentrations, wherein the ratio was 92% at 3.33 ppm, 83% at 11.1 ppm, and 30% at 33.3 ppm. FIG. 1D indicates the melanin content ratio after adding 4-cis-D-hydroxyproline at various concentrations, wherein the ratio was 100% at 3.33 ppm, 98% at 11.1 ppm, 96% at 33.3 ppm, and 32% at 333 ppm. From the results above, it was found that 4-trans-L-hydroxyproline and 4-trans-D-hydroxyproline had no effect on reducing melanin content. On the other hand, 4-cis-L-hydroxyproline and 4-cis-D-hydroxyproline were found to have an effect on reducing melanin content in a concentration-dependent manner.

### Experiments for quantifying cell growth and melanin content by using a mixture solution of 4-trans-L-hydroxyproline and 4-cis-D-hydroxyproline

The equal amounts of 4-trans-L-hydroxyproline and 4-cis-D-hydroxyproline were mixed to produce the mixture solution of 4-trans-L-hydroxyproline and 4-cis-D-hydroxyproline, which was then provided for the experiments. Cell culture and quantification and evaluation of number of the cells and melanin were carried out in the same manner as the Examples above.

### Results of cell growth test using mixture solution

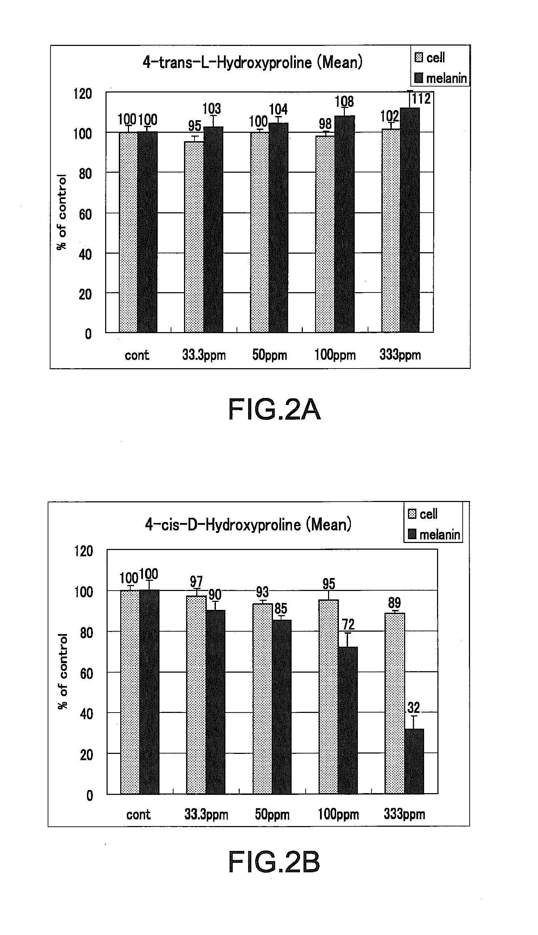

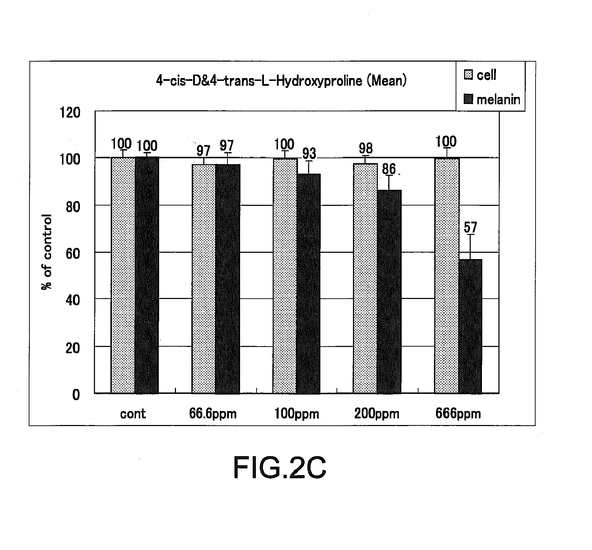

In Figs. 2A to 2C (hatched bars), the results for investigating cell growth of mouse B16 melanoma cells are shown, wherein 4-trans-L-hydroxyproline and 4-cis-D-hydroxyproline as well as their mixture were added at various concentrations to the cells. The error bar for each experiment condition indicates standard deviation of test results that were measured in duplicate under the same condition. Fig. 2A illustrates the number of cells after adding 4-trans-L-hydroxyproline at various concentrations when the control was represented as 100%. It was 95% at 33.3 ppm, 100% at 50 ppm, 98% at 100 ppm, and 102% at 333 ppm. Fig. 2B illustrates the number of cells after adding 4-cis-L-hydroxyproline at various concentrations when the control was 100%. It was 97% at 33.3 ppm, 93% at 50 ppm, 95% at 100 ppm, and 89% at 333 ppm. Fig. 2C illustrates the number of cells after adding the mixture of the compounds at various concentrations when the control was represented as 100%. It was 97% at 66.6 ppm, 100% at 100 ppm, 98% at 200 ppm, and 100% at 666 ppm. From the results above, the number of cells when the control was represented as 100% was not different if the concentration of the mixture containing 4-trans-L-hydroxyproline and 4-cis-D-hydroxyproline was between 66.6 ppm and 666 ppm, and thus it became evident that the mixture had no effect on cell growth of mouse B16 melanoma cells.

### Results of quantifying melanin using the mixture solution

In FIGS. 2A to 2C (solid bars), the results for investigating the effect on reducing melanin content in mouse B16 melanoma cells are shown, wherein 4-trans-L-hydroxyproline and 4-cis-D-hydroxyproline as well as their mixture were added at various concentrations to the cells. The error bar for each test condition indicates standard deviation of measured values of test results that were measured in duplicate under the same condition. FIG. 2A illustrates the melanin content ratio after adding 4-trans-L-hydroxyproline at various concentrations. It was 103% at 33.3 ppm, 104% at 50 ppm, 108% at 100 ppm, and 112% at 333 ppm. FIG. 2B illustrates the melanin content ratio after adding 4-cis-D-hydroxyproline at various concentrations. It was 90% at 33.3 ppm, 85% at 50 ppm, 72% at 100 ppm, and 32% at 333 ppm. FIG. 2C illustrates the melanin content ratio after adding the mixture at various concentrations. It was 97% at 66.6 ppm, 93% at 100 ppm, 86% at 200 ppm, and 57% at 666 ppm. From the results above, it was found that 4-cis-D-hydroxyproline could prominently inhibit melanin synthesis in a concentration-dependent manner starting from 33.3 ppm. However, in a case in which the mixture of the two was added, no prominent inhibition of melanin content was observed even though the same amount was added as the case in which only 4-cis-D-hydroxyproline was added.

### Test for determining cell growth and melanin content reduction using a mixture at various mixing ratio

By using the mixture solution in which 4-trans-L-hydroxyproline and 4-cis-D-hydroxyproline were mixed at various ratio, test for determining cell growth and melanin content reduction was carried out. Cell culture and quantification and evaluation were carried out in the same manner as the Examples above.

### Results of cell growth test using the mixture solution at various mixing ratio

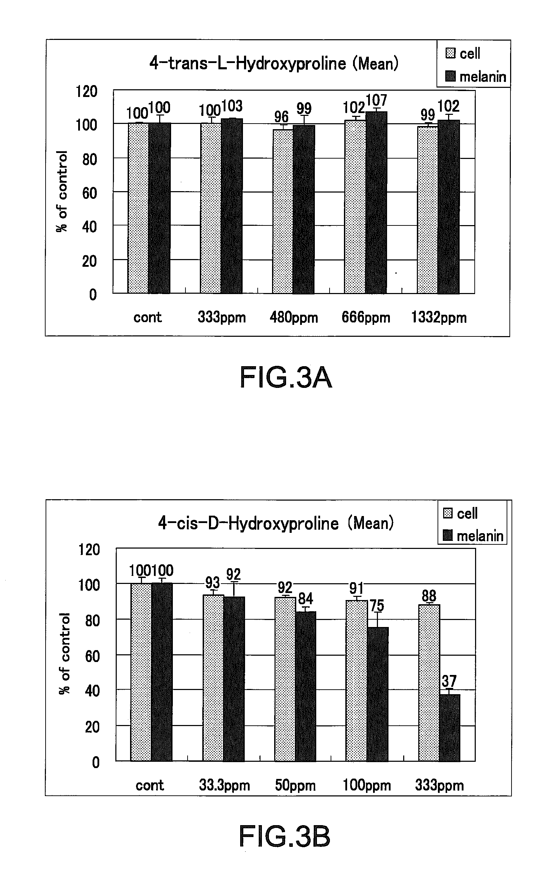

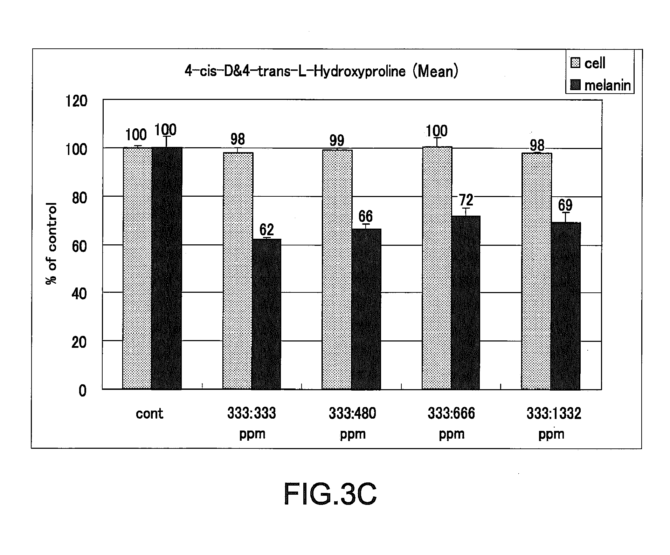

In FIG. 3 (hatched bar), test results for investigating cell growth of mouse B16 melanoma cells are illustrated, wherein 4-trans-L-hydroxyproline and 4-cis-D-hydroxyproline as well as their mixture were added at various concentrations to the cells. The error bar for each test condition indicates standard deviation of measured values of test results that were measured in duplicate under the same condition. FIG. 3A illustrates the number of cells after adding 4-trans-L-hydroxyproline at various concentrations when the control was represented as 100%. It was 100% at 333 ppm, 96% at 480 ppm, 102% at 666 ppm, and 99% at 1332 ppm. FIG. 3B illustrates the number of cells after adding 4-cis-D-hydroxyproline at various concentrations when the control was represented as 100%. It was 93% at 33.3 ppm, 92% at 50 ppm, 91% at 100 ppm, and 88% at 333 ppm. FIG. 3C illustrates the number of cells after adding the mixture of 4-trans-L-hydroxyproline and 4-cis-D-hydroxyproline (4-cis-D : 4-trans-L) at various concentrations wherein the compounds were mixed with different mixing ratio and the control was represented as 100%. It was 98% at 333 ppm : 333pm, 99% at 333 ppm : 480 ppm, 100% at 333 ppm : 666 ppm, and 98% at 333 ppm : 1332 ppm. Addition of the mixture having 4-cis-D-hydroxyproline and 4-trans-L-hydroxyproline exhibited the same number of cells as the control when the control was represented as 100%.

### Results of melanin quantification using the mixture solution at various mixing ratio

In FIG. 3 (solid bar), measured values of the results for investigating the effect of reducing melanin content in mouse B16 melanoma cells are illustrated, wherein 4-trans-L-hydroxyproline and 4-cis-D-hydroxyproline as well as their mixture were added at various concentrations to the cells. The error bar for each test condition indicates standard deviation of measured values of test results that were measured in duplicate under the same condition. FIG. 3A illustrates the melanin content ratio after adding 4-trans-L-hydroxyproline at various concentrations. It was 103% at 333 ppm, 99% at 480 ppm, 107% at 666 ppm, and 102% at 1332 ppm. FIG. 3B illustrates the melanin content ratio after adding 4-cis-D-hydroxyproline at various concentrations. It was 92% at 33.3 ppm, 84% at 50 ppm, 75% at 100 ppm, and 37% at 333 ppm. FIG. 3C illustrates the melanin content ratio after adding the mixture of 4-trans-L-hydroxyproline and 4-cis-D-hydroxyproline (4-cis-D : 4-trans-L) at various concentrations wherein the compounds were mixed with different mixing ratio. It was 62% at 333 ppm : 333pm, 66% at 333 ppm : 480 ppm, 72% at 333 ppm : 666 ppm, and 69% at 333 ppm : 1332 ppm. From the results above, it was found that the addition of the mixture having 4-cis-D-hydroxyproline and 4-trans-L-hydroxyproline could inhibit the melanin content in a concentration-dependent manner that has been shown in the case in which 4-cis-D-hydroxyproline was added alone. However, no prominent reduction of melanin content was obtained even when the concentration of 4-trans-L-hydroxyproline had been increased compared to 4-cis-D-hydroxyproline.

Mix examples including an emulsion formulation, a skin patch, a tablet, a soft capsule, a granule, a drink, a candy, a cookie, soybean paste, a French dressing, mayonnaise, baguette, soy sauce, furikake (a seasoned powder for sprinkling over rice), seasoning andnatto (Japanese fermented soybean paste) sauce, natto, non-filtered dark vinegar, a cream, a body cream, a gel formulation, a peel-off mask, an impregnation mask, an emulsion, a cosmetic water, and an aerosol, all containing 4-cis-hydroxyproline according to the invention, are given below. However, the mix examples are listed only for illustration and it is not intended that the scope of the invention is limited by them.

### Mix example 1 (emulsion formulation)

| (Composition) | Addition amount (% by weight) |
|---|---|
| 4-cis-hydroxyproline | 0.42 |
| behenyl alcohol | 0.2 |
| cetanol | 0.5 |
| glycerin monofatty acid ester | 1.8 |
| hydrogenated castor oil POE (60) | 1.0 |
| white vaseline | 2.0 |
| fluid paraffin | 10.0 |
| isopropyl myristate | 3.0 |
| methyl polysiloxane (6 cs) | 1.5 |
| conc. glycerin | 13.0 |
| dipropylene glycol | 2.0 |
| carboxyvinyl polymer | 0.25 |
| sodium hyaluronic acid | 0.005 |
| potassium hydroxide | q.s. |
| lactic acid | q.s. |
| sodium edetate | q.s. |
| ethyl paraben | q.s. |
| purified water | remainder |
| | 100.000 |

### Mix example 2 (skin patch)

| (Composition) | Addition amount (% by weight) |
|---|---|
| 4-cis-hydroxyproline | 0.3 |
| polyacrylic acid | 3.0 |
| sodium polyacrylate | 2.5 |
| gelatin | 0.5 |
| sodium carboxymethyl cellulose | 4.0 |
| polyvinyl alcohol | 0.3 |
| conc. glycerin | 14.0 |
| 1,3-butylene glycol | 12.0 |
| aluminum hydroxide | 0.1 |
| sodium edetate | 0.03 |
| methyl paraben | 0.1 |
| purified water | remainder |
| | 100.00 |

### Mix example 3 (tablet)

| (Composition) | Addition amount (in mg/tablet) |
|---|---|
| 4-cis-hydroxyproline | 360.5 |
| lactose | 102.4 |
| calcium carboxymethyl | 29.9 |
| cellulose | |
| hydroxypropyl cellulose | 6.8 |
| magnesium stearate | 5.2 |
| crystalline cellulose | 10.2 |
| | 515.0 |

| | |
|---|---|
| Mix example 4 (tablet) | |

| (Composition) | Addition amount (in mg/tablet) |
|---|---|
| sucrose ester | 70 |
| crystalline cellulose | 74 |
| methyl cellulose | 36 |
| glycerin | 25 |
| 4-cis-hydroxyproline | 475 |
| N-acetyl glucosamine | 200 |
| hyaluronic acid | 150 |
| vitamin E | 30 |
| vitamin B6 | 20 |
| vitamin B2 | 10 |
| α-lipoic acid | 20 |
| coenzyme Q10 | 40 |
| ceramide (devil's tongue jelly | 50 |
| extract) | |
| L-proline | 300 |
| | 1500 |

### Mix example 5 (soft capsule)

| (Composition) | Addition amount (in mg/capsule) |
|---|---|
| soybean oil for cooking | 530 |
| eucommia bark extract | 50 |
| ginseng extract | 50 |
| 4-cis-hydroxyproline | 100 |
| royal jelly | 50 |
| maca | 30 |
| GABA | 30 |
| beeswax | 60 |
| gelatin | 375 |
| glycerin | 120 |
| glycerin fatty acid ester | 105 |
| | 1500 |

### Mix example 6 (soft capsule)

| (Composition) | Addition amount (in mg/capsule) |
|---|---|
| brown rice germ oil | 659 |
| 4-cis-hydroxyproline | 500 |
| resveratrol | 1 |
| lotus germ extract | 100 |
| elastin | 180 |
| DNA | 30 |
| folic acid | 30 |
| | 1500 |

### Mix example 7 (granule)

| (Composition) | Addition amount (in mg/pack) |
|---|---|
| 4-cis-hydroxyproline | 400 |
| vitamin C | 100 |
| soybean isoflavone | 250 |
| reduced lactose | 300 |
| soybean oligosugar | 36 |
| erythritol | 36 |
| dextrin | 30 |
| flavor | 24 |
| citric acid | 24 |
| | 1200 |

### Mix example 8 (drink)

| (Composition) | Addition amount (in g/60 mL) |
|---|---|
| eucommia bark extract | 1.6 |
| ginseng extract | 1.6 |
| 4-cis-hydroxyproline | 1.6 |
| reduced maltose syrup | 28 |
| erythritol | 8 |
| citric acid | 2 |
| flavor | 1.3 |
| N-acetyl glucosamine | 1 |
| hyaluronic acid Na | 0.5 |
| vitamin E | 0.3 |
| vitamin B6 | 0.2 |
| vitamin B2 | 0.1 |
| α-lipoic acid | 0.2 |
| coenzyme Q10 | 1.2 |
| ceramide (devil' s tongue jelly extract) | 0.4 |
| L-proline | 2 |
| purified water | remainder |
| | 60 |

### Mix example 9 (candy)

| (Composition) | Addition amount (% by weight) |
|---|---|
| sugar | 50 |
| syrup | 48 |
| 4-cis-hydroxyproline | 1 |
| flavor | 1 |
| | 100 |

### Mix example 10 (cookie)

| (Composition) | Addition amount (% by weight) |
|---|---|
| soft flour | 45.0 |
| butter | 17.5 |
| granulated sugar | 20.0 |
| 4-cis-hydroxyproline | 4.0 |
| egg | 12.5 |
| flavor | 1.0 |
| | 100.0 |

### Method for preparation of mix example 10

### (cookie)

Granulated sugar is gradually added to butter under mixing. Egg, decapeptide, GABA, and the flavor are further added and mixed. After mixing thoroughly, soft flour obtained by uniformly sieving is added to the mixture, which is then mixed at low speed. The agglomerated mixture is kept in a refrigerator. After molding, cookies are obtained by baking for 15 minutes at 170°C.

### Mix example 11 (soybean paste)

| (Composition) | Addition amount (g) |
|---|---|
| soybean | 1000 |
| rice yeast | 1000 |
| salt | 420 |
| 4-cis-hydroxyproline | 158 |
| water | remainder |
| | 4000 |

### Method for preparation of mix example 11 (soybean paste)

Rice yeast and salt are mixed well with each other. Washed soybeans are soaked in water (x3 volume) overnight. Water is removed, and the soybeans are cooked while adding fresh water and then placed in a basket. The cooking water (i.e. filtered water) is collected and 4-cis-hydroxyproline is dissolved therein to 10% w/v. The cooked soybeans are immediately smashed and added with the rice yeast mixed with salt. While adding thereto the filtered water in which 4-cis-hydroxyproline is dissolved, the beans are thoroughly mixed until clay-such as hardness is obtained. The mixture is rolled to have a meat ball shape, and the resultant is filled in a container from end to end. After making the surface flat, the container is sealed by covering with a plastic wrap. The container is changed three months later, and again, after making the surface flat, the container is sealed by covering with a plastic wrap. Meanwhile, in addition to adding 4-cis-hydroxyproline to filtered water, rice yeast which can produce a large amount of 4-cis-hydroxyproline can also be used. In addition, 4-cis-hydroxyproline or its salt can be added to commercially available soybean paste.

### Mix example 12 (French dressing)

| (Composition) | Addition amount (g) |
|---|---|
| salad oil | 27.0 |
| vinegar | 30.0 |
| sodium chloride | 0.9 |
| 4-cis-hydroxyproline | 1.1 |
| pepper | 1.0 |
| | 60.0 |

### Method for preparation of mix example 12 (French dressing)

To vinegar, sodium chloride and 4-cis-hydroxyproline are added and stirred well for dissolution. Salad oil is added thereto and pepper is added under thorough mixing.

### Mix example 13 (mayonnaise)

| (Composition) | Addition amount (g) |
|---|---|
| salad oil | 134.0 |
| vinegar | 5 |
| sodium chloride | 0.9 |
| 4-cis-hydroxyproline | 1 |
| egg yolk | 18 |
| sugar | 0.2 |
| pepper | 0.9 |
| | 160.0 |

### Method for preparation of mix example 13

### (mayonnaise)

To egg yolk (room temperature), vinegar, sodium chloride, 4-cis-hydroxyproline, and pepper are added and the mixed sufficiently by using a whipping machine. The mixing is further continued while adding salad oil in small portions to obtain an emulsion. Finally, sugar is added and mixed.

### Mix example 14 (baguette)

| (Composition) | Addition amount (g) |
|---|---|
| strong flour | 140 |
| soft flour | 60 |
| sodium chloride | 3 |
| sugar | 6 |
| 4-cis-hydroxyproline | 2 |
| dry yeast | 4 |
| warm water | 128 |
| | 343 |

### Method for preparation of mix example 14 (baguette)

To warm water, 1 g of sugar and dry yeast are added for pre-fermentation. Strong flour, soft flour, sodium chloride, 5 g of sugar, and 4-cis-hydroxyproline are added to a bowl, and the pre-fermented yeast is added thereto. After sufficient kneading, the mixture is formed into a ball, and subjected to primary fermentation at 30°C. The dough is kneaded again, kept for a while, and then formed into an appropriate shape. Final fermentation is carried out by using an electronic fermentation machine. After forming coupes, baking is carried out in an oven at 220°C for 30 minutes.

### Mix example 15 (soy sauce)

| (Composition) | Addition amount (g) |
|---|---|
| commercially available soy sauce | 990 |
| 4-cis-hydroxyproline | 10 |
| | 1000 |

### Method for preparation of mix example 15 (soy sauce)

To commercially available soy sauce, 4-cis-hydroxyproline is added and stirred well. Meanwhile, in addition to adding 4-cis-hydroxyproline or its salt, soy sauce can be produced by using yeast which can produce a large amount of 4-cis-hydroxyproline.

### Mix example 16 (yogurt)

| (Composition) | Addition amount (g) |
|---|---|
| milk | 880 |
| L. Bulgaricus | 50 |
| S. Thermophilus | 50 |
| 4-cis-hydroxyproline | 20 |
| | 1000 |

### Method for preparation of mix example 16 (yogurt)

Fermentation is carried out at 40°C to 45°C. Other commercially available starter cultures can be used and also 4-cis-hydroxyproline can be added to commercially available yogurt. In addition, instead of adding 4-cis-hydroxyproline or its salt, a microorganism which can produce a large amount of 4-cis-hydroxyproline can be used.

### Mix example 17 (furikake)

| (Composition) | Addition amount (g) |
|---|---|
| 4-cis-hydroxyproline | 50 |
| seaweed | 15 |
| sodium L-glutamate | 10 |
| sodium chloride | 2 |
| roasted sesame seed | 10 |
| thinly sliced dried mackerel | 10 |
| sugar | 1 |
| soy sauce | 2 |
| | 100 |

### Mix example 18 (seasoning and natto sauce)

| (Composition) | Addition amount (g) |
|---|---|
| commercially available natto sauce | 9.9 |
| 4-cis-hydroxyproline | 0.1 |
| | 10 |

### Mix example 19 (natto)

| (Composition) | Addition amount (g) |
|---|---|
| commercially available natto | 19.9 |
| 4-cis-hydroxyproline | 0.1 |
| | 20 |

### Method for preparation of mix example 19 (natto)

Instead of adding 4-cis-hydroxyproline or its salt, a microorganism which can produce a large amount of 4-cis-D-hydroxyproline can be used for producing natto.

### Mix example 20 (non-filtered dark vinegar)

| (Composition) | Addition amount (g) |
|---|---|
| commercially available non-filtered dark vinegar | 950 |
| 4-cis-hydroxyproline | 50 |
| | 1000 |

### Method for preparation of mix example 20 (non-filtered dark vinegar)

Instead of adding 4-cis-hydroxyproline or its salt, a microorganism which can produce a large amount of 4-cis-D-hydroxyproline can be used for producing vinegar, dark vinegar, and non-filtered dark vinegar.

### Mix example 21 (cream)

| (Composition) | Addition amount (%) |
|---|---|
| fluid paraffin | 3 |
| vaseline | 1 |
| dimethyl polysiloxane | 1 |
| stearyl alcohol | 1.8 |
| behenyl alcohol | 1.6 |
| glycerin | 8 |
| dipropylene glycol | 5 |
| macadamia nut oil | 2 |
| hydrogenated oil | 3 |
| squalene | 6 |
| stearic acid | 2 |
| cholesteryl hydroxystearate | 0.5 |
| cetyl 2-ethylhexanoate | 4 |
| polyoxyethylene hydrogenated | 0.5 |
| castor oil | |
| self-emulsifying glycerin | 3 |
| monostearate | |
| potassium hydroxide | 0.15 |
| sodium hexametaphosphate | 0.05 |
| trimethyl glycine | 2 |
| α-tocopherol 2-L-ascorbic acid | |
| phosphoric acid diester | 1 |
| potassium | |
| tocopherol acetate | 0.1 |
| 4-cis-hydroxyproline | 4 |
| paraben | q.s. |
| trisodium edetate | 0.05 |
| 4-t-butyl-4'-methoxy | 0.05 |
| dibenzoylmethane | |
| diparamethoxy cinnamic acid | |
| mono-2-ethylhexanoic acid | 0.05 |
| glyceryl | |
| coloring agent | q.s. |
| carboxyvinyl polymer | 0.05 |
| purified water | remainder |
| | 100.00 |

### Mix example 22 (body cream)

| (Composition) | Addition amount (%) |
|---|---|
| dimethyl polysiloxane | 3 |
| decamethyl cyclopentasiloxane | 13 |
| dodecamethyl cyclohexasiloxane | 12 |
| polyoxyethylene-methyl | 1 |
| polysiloxane copolymer ethanol | 2 |
| isopropanol | 1 |
| glycerin | 3 |
| dipropylene glycol | 5 |
| polyethylene glycol 6000 | 5 |
| sodium hexametaphosphate | 0.05 |
| tocopherol acetate | 0.1 |
| 4-cis-hydroxyproline | 5 |
| fennel extract | 0.1 |
| hammamelis extract | 0.1 |
| ginseng extract | 0.1 |
| L-menthol | q.s. |
| paraoxybenzoic acid ester | q.s. |
| trisodium edetate | 0.05 |
| dimorpholino pyridazinone | 0.01 |
| methylbis(trimethylsiolxy)silyl | 0.1 |
| isopentyl trimethoxycinnamate | |
| iron sulfate | q.s. |
| cobalt titanate | q.s. |
| dimethyl distearyl ammonium | 1.5 |
| hectorite | |
| polyvinyl alcohol | 0.1 |
| hydroxyethyl cellulose | 0.1 |
| trimethylsiloxy silicate | 2 |
| flavor | q.s. |
| purified water | remainder |
| | 100.00 |

### Mix example 23 (gel formulation)

| (Composition) | Addition amount (%) |
|---|---|
| dimethyl polysiloxane | 5 |
| glycerin | 2 |
| 1,3-butylene glycol | 5 |
| polyethylene glycol 1500 | 3 |
| polyethylene glycol 20000 | 3 |
| cetyl octanoate | 3 |
| citric acid | 0.01 |
| sodium citrate | 0.1 |
| sodium hexametaphosphate | 0.1 |
| dipotassium glycyrrhiziate | 0.1 |
| 4-cis-hydroxyproline | 2 |
| tocopherol acetate | 0.1 |
| skullcap extract | 0.1 |
| strawberry geranium extract | 0.1 |
| trisodium edetate | 0.1 |
| xanthan gum | 0.3 |
| acrylate-alkyl methacrylate | 0.05 |
| copolymer (PEMULEN TR-2) | |
| agar powder | 1.5 |
| phenoxyethanol | q.s. |
| dibutylhydroxy toluene | q.s. |
| purified water | remainder |
| | 100.00 |

### Mix example 24 (peel-off mask)

| (Composition) | Addition amount (%) |
|---|---|
| ethanol | 10 |
| 1,3-butylene glycol | 6 |
| polyethylene glycol 4000 | 2 |
| olive oil | 1 |
| macadamia nut oil | 1 |
| phytosteryl hydroxystearate | 0.05 |
| lactic acid | 0.05 |
| sodium lactate | 0.1 |
| L-ascorbic acid disodium sulfuric | 0.1 |
| acid ester | |
| α-tocopherol 2-L-ascorbic acid | 0.1 |
| phosphoric acid diester potassium | |
| 4-cis-hydroxyproline | 10 |
| fish collagen | 0.1 |
| chondroitin sodium sulfate | 0.1 |
| sodium carboxymethyl cellulose | 0.2 |
| polyvinyl alcohol | 12 |
| paraoxy benzoic acid ester | q.s. |
| flavor | q.s. |
| purified water | remainder |
| | 100.00 |

### Mix example 25 (impregnation mask)

| (Composition) | Addition amount (%) |
|---|---|
| glycerin | 1 |
| 1,3-butylene glycol | 8 |
| xylit | 2 |
| polyethylene glycol 1500 | 2 |
| rosemary oil | 0.01 |
| sage oil | 0.1 |
| citric acid | 0.02 |
| sodium citrate | 0.08 |
| sodium hexametaphosphate | 0.01 |
| hydroxypropyl-β-cyclodextrin | 0.1 |
| 4-cis-hydroxyproline | 0.5 |
| birch extract | 0.1 |
| lavender oil | 0.01 |
| xanthan gum | 0.05 |
| carboxyvinyl polymer | 0.15 |
| paraoxy benzoic acid ester | q.s. |
| purified water | remainder |
| | 100.00 |

### Mix example 26 (emulsion)

| (Composition) | Addition amount (%) |
|---|---|
| fluid paraffin | 7 |
| vaseline | 3 |
| decamethyl cyclopentasiloxane | 2 |
| behenyl alcohol | 1.5 |
| glycerin | 5 |
| dipropylene glycol | 7 |
| polyethylene glycol 1500 | 2 |
| jojoba oil | 1 |
| isostearic acid | 0.5 |
| stearic acid | 0.5 |
| behenic acid | 0.5 |
| tetra 2-ethylhexanoic acid | 3 |
| pentaerythritol | |
| cetyl 2-ethylhexanoate | 3 |
| monostearic acid glycerin | 1 |
| monostearic acid polyoxyethylene | 1 |
| glycerin | |
| potassium hydroxide | 0.1 |
| sodium hexametaphosphate | 0.05 |
| stearyl glycyrrhetate | 0.05 |
| 4-cis-hydroxyproline | 1 |
| royal jelly extract | 0.1 |
| yeast extract | 0.1 |
| tocopherol acetate | 0.1 |
| sodium acetylated hyaluronic acid | 0.1 |
| trisodium edetate | 0.05 |
| 4-t-butyl-4'-methoxy dibenzoylmethane | 0.1 |
| 2-ethylhexyl paramethoxycinnamate | 0.1 |
| carboxyvinyl polymer | 0.15 |
| paraben | q.s. |
| flavor | q.s. |
| purified water | remainder |
| | 100.00 |

### Mix example 27 (emulsion)

| (Composition) | Addition amount (%) |
|---|---|
| dimethyl polysiloxane | 2 |
| behenyl alcohol | 1 |
| batyl alcohol | 0.5 |
| glycerin | 5 |
| 1,3-butylene glycol | 7 |
| erythritol | 2 |
| hydrogenated oil | 3 |
| squalene | 6 |
| tetra-2-ethylhexanoic acid | 2 |
| pentaerythritol | |
| isostearic acid | 1 |
| polyoxyethylene glyceryl | |
| monostearic acid | 1 |
| polyoxyethylene glycerin | |
| 4-cis-hydroxyproline | 0.3 |
| potassium hydroxide | q.s. |
| sodium hexametaphosphate | 0.05 |
| phenoxyethanol | q.s. |
| carboxyvinyl polymer | 0.1 |
| purified water | remainder |
| | 100.00 |

### Mix example 28 (cosmetic water)

| (Composition) | Addition amount (%) |
|---|---|
| ethyl alcohol | 5 |
| glycerin | 1 |
| 1,3-butylene glycol | 5 |
| polyoxyethylene | |
| polyoxypropylene decyl | 0.2 |
| tetradecyl ether | |
| sodium hexametaphosphate | 0.03 |
| trimethyl glycine | 1 |
| sodium polyasparaginic acid | 0.1 |
| α-tocopherol 2-L-ascorbic acid | |
| phosphoric acid diester | 0.1 |
| potassium | |
| thiotaurine | 0.1 |
| 4-cis-hydroxyproline | 8 |
| trisodium EDTA | 0.1 |
| carboxyvinyl polymer | 0.05 |
| potassium hydroxide | 0.02 |
| phenoxyethanol | q.s. |
| flavor | q.s. |
| purified water | remainder |
| | 100.00 |

### Mix example 29 (cosmetic water)

| (Composition) | Addition amount (%) |
|---|---|
| ethanol | 10 |
| dipropylene glycol | 1 |
| polyethylene glycol 1000 | 1 |
| polyoxyethylene | 1 |
| methylglucoside | |
| jojoba oil | 0.01 |
| tri-2-ethylhexanoic acid | 0.1 |
| glyceryl | |
| polyoxyethylene hydrogenated | 0.2 |
| castor oil | |
| diisostearic acid polyglyceryl | 0.15 |
| sodium N-stearoyl-L-glutamate | 0.1 |
| citric acid | 0.05 |
| sodium citrate | 0.2 |
| potassium hydroxide | 0.4 |
| dipotassium glycyrrhiziate | 0.1 |
| arginine hydrochloride | 0.1 |
| L-ascorbic acid 2-glucoside | 2 |
| 4-cis-hydroxyproline | 0.5 |
| trisodium edetate | 0.05 |
| 2-ethylhexyl paramethoxy | 0.01 |
| cinnamate | |
| dibutylhydroxy toluene | q.s. |
| paraben | q.s. |
| deep sea water | 3 |
| flavor | q.s. |
| purified water | remainder |
| | 100.00 |

### Mix example 30 (urea aerosol stock solution for external application)

| (Composition) | Addition amount (% by weight) |
|---|---|
| ethanol | 15.0 |
| polyoxyethylene hydrogenated | |
| castor oil 50 | 1.5 |
| diphenhydramine | 1.0 |
| dibucaine | 2.0 |
| tocopherol acetate | 0.5 |
| 4-cis-hydroxyproline | 0.1 |
| isostearic acid | 0.1 |
| 1,3-butylene glycol | 3.0 |
| polyethylene glycol 400 | 3.0 |
| camphor | 0.05 |
| urea | 20.0 |
| purified water | remainder |
| | 100.00 |

### Mix example 31 (aerosol urea spraying agent)

| (Composition) | Addition amount (% by weight) |
|---|---|
| urea aerosol stock | 65.0 |
| solution for external | |
| application | |
| dimethyl ether | 35.0 |
| | 100.00 |

### Method of filling mix example 31 (aerosol urea spraying agent)

An aerosol urea stock solution for external application and dimethyl ether are filled in a pressure-resistant aerosol aluminum container having an inner surface coated with Teflon (registered trademark) to produce an aerosol formulation.

## Claims

1. A skin-whitening agent comprising one or more compounds selected from the group consisting of 4-cis-hydroxyproline and a derivative and/or a salt thereof.

2. The skin-whitening agent according to claim 1, wherein the 4-cis-hydroxyproline is 4-cis-L-hydroxyproline.

3. The skin-whitening agent according to claim 1, wherein the 4-cis-hydroxyproline is 4-cis-D-hydroxyproline.

4. The skin-whitening agent according to claims 1 to 3, wherein the agent is an oral agent.

5. A skin-whitening composition for external formulation to skin comprising the skin-whitening agent according to any one of claims 1 to 3.

6. A food composition comprising the skin-whitening agent according to any one of claims 1 to 3.

7. A pharmaceutical composition comprising the skin-whitening agent according to any one of claims 1 to 3.

8. A composition for external formulation to skin comprising one or more compounds selected from the group consisting of 4-cis-hydroxyproline and a derivative and/or a salt thereof.

9. A cosmetic method for whitening skin comprising a step of administering one or more compounds selected from the group consisting of 4-cis-hydroxyproline and a derivative and/or a salt thereof.

10. The method according to claim 9, wherein the 4-cis-hydroxyproline is 4-cis-L-hydroxyproline.

11. The method according to claim 9, wherein the 4-cis-hydroxyproline is 4-cis-D-hydroxyproline.
